# EUROPEAN PATENT SPECIFICATION

(11) **EP 3 735 837 B1**
(45) Date of publication and mention of the grant of the patent: **25.06.2025**
(21) Application number: 19754685.6
(22) Date of filing: 14.02.2019
(51) Int. Cl.: A23K 10/12, A23K 30/00, C12N 1/16, C12N 1/20, C12R 1/07, C12R 1/865, A23K 10/16, A23K 10/18, A23K 10/30, C12N 1/18

(54) **METHOD FOR PREPARING FERMENTED COMPOSITION WITH IMPROVED ODOR USING YEAST**
VERFAHREN ZUR HERSTELLUNG EINER FERMENTIERTEN ZUSAMMENSETZUNG MIT VERBESSERTEM GERUCH UNTER VERWENDUNG VON HEFE
PROCÉDÉ DE PRÉPARATION D'UNE COMPOSITION FERMENTÉE AYANT UNE ODEUR AMÉLIORÉE AU MOYEN D'UNE LEVURE

(30) Priority: 14.02.2018 KR 20180018449
(43) Date of publication of application: 11.11.2020
(73) Proprietor: CJ Cheiljedang Corporation, Seoul 04560 (KR)
(72) Inventor: SEO, HyoJeong, Seoul 04560 (KR); YANG, Tae Joo, Seoul 04560 (KR); CHI, Hyun, Seoul 04560 (KR); CHOI, Myeong-hyeon, Seoul 04560 (KR); PARK, Seung Won, Seoul 04560 (KR); HONG, YoungHo, Seoul 04560 (KR)
(74) Representative: Icosa
(86) International application number: PCT/KR2019/001841
(87) International publication number: WO 2019/160365

(56) References cited:
- CN-A- 103 027 187
- CN-A- 105 410 337
- CN-A- 107 232 393
- KR-A- 20130 050 796
- KR-A- 20140 144 329
- KR-A- 20170 037 483
- KR-B1- 100 753 457
- US-A1- 2013 274 443
- CHI CHUN-HUA ET AL: "Improvement of bioactivity of soybean meal by solid-state fermentation withBacillus amyloliquefaciensversusLactobacillusspp. andSaccharomyces cerevisiae", LWT- FOOD SCIENCE AND TECHNOLOGY, ACADEMIC PRESS, UNITED KINGDOM, vol. 68, 9 December 2015 (2015-12-09), pages 619 - 625, XP029404877, ISSN: 0023-6438, DOI: 10.1016/J.LWT.2015.12.002
- FERNANDEZ-LEIRO, R: "Structural Analysis of Saccharomyces cerevisiae a-Galactosidase and Its Complexes with Natural Substrates Reveals New Insights into Substrate Specificity of GH27 Glycosidases", THE JOURNAL OF BIOLOGICAL CHEMISTRY, vol. 285, no. 363, 2010, pages 28020 - 28033, XP055632790
- MOEHLE, C. M.: "Protease B of Saccharomyces cerevisiae: Isolation and Re- gulation of the PRBI Structural Gene", GENETICS, vol. 115, February 1987 (1987-02-01), pages 255 - 263, XP055632794
- VEIDE, J: "Improved extracellular phytase activity in Saccharomyces cere- visiae by modifications in the PHO system", INTERNATIONAL JOURNAL OF FOOD MICROBIOLOGY, vol. 108, 2006, pages 60 - 67, XP024956458

## Description

### TECHNICAL FIELD

The present invention relates to a method for preparing a fermented composition, and more specifically, to a method for preparing a fermented composition with improved odor, which includes preparing grain flour, performing primary fermentation of the grain flour using yeast which produces α-galactosidase, protease, and phytase; performing secondary fermentation of the primary fermented product using a strain of the genus *Bacillus,* and obtaining the secondary fermented product; and use of yeast producing α-galactosidase, protease, and phytase for improving the odor of a fermented product of *Bacillus.*

### BACKGROUND ART

Grain is widely used as feed for livestock because of high feed efficiency due to its high energy content and good digestibility due to its low crude fiber content. However, grain feed has a low content of proteins and amino acids, and thus an auxiliary supplement is essentially required for balanced nutrition. As such protein sources, animal protein sources (*e.g.,* fish meal, powdered skim milk, meat meal, blood, *etc.*) and plant protein sources (*e.g.,* soybean, seeds, flax, *etc.*) are used. Corn gluten, which is a plant protein source, is a by-product of corn starch preparation that is similar in content to fish meal with a high protein content (about 3 times that of common plant protein sources) and low in price, and is thus widely used as a protein source for feed. Additionally, soybean meal, which is a by-product that remains after the production of soybean oil from soybeans, is used as a major source of proteins in feed due to its high protein content.

However, indigestible oligosaccharides, non-soluble proteins modified during the manufacturing process, *etc.* contained in the soybean meal or corn gluten decrease the digestibility and are thus problematic for its use as feed. Therefore, there is a need for the development of a novel processing method capable of improving the digestibility of the protein parts so that the soybean meal or corn gluten can be used as high-quality protein feed.

Additionally, in this regard, various complex microbial agents which can be used for a feed composition have recently been developed, but these complex microbial agents have a problem in that the physiological actions and interactions among microorganisms have not been considered.

Under the circumstances, the present inventors have made efforts to solve the above problems. As a result, they have discovered that it is possible to increase the water-soluble saccharide content of a raw material via enzyme treatment of grain flour and to concentrate the proteins contained therein via yeast fermentation with yeasts producing α-galactosidase, protease, and phytase and *Bacillus* fermentation, and thus it is possible to improve functionality of a composition by increasing the proportion of the protein content and the viable cell count in the grain flour, as well as its odor, thereby completing the present disclosure.

The prior art describes methods for preparing fermented compositions comprising a step of performing fermentation with yeasts and a step of performing fermentation with a strain of the genus *Bacillus,* as well as methods for concentrating proteins in grain powder.

As an example, CN105410337 discloses, in embodiment 4, a method for preparing a fermented composition, wherein soybean meal and corn starch are fermented with yeast extract, inoculated with *Bacillus subtilis* and further fermented.

CN107232393 teaches, in Example 2, a method for preparing a cotton meal protein feed, wherein dry cotton meal is fermented with active dry yeast for wine, inoculated with *Bacillus subtilis* and further fermented.

CN103027187 discloses, in Example 1, a method for preparing a fermented protein feed, wherein soybean meal and cotton meal are fermented with *Bacillus,* then inoculated with a mixture of *Saccharomyces cerevisiae* and *Pediococcus acidilactici* and further fermented.

KR20170037483 teaches methods for increasing protein content in corn gluten, wherein corn gluten is fermented with yeasts (Example 9), or with a strain of the genus *Bacillus* (Example 5).

In addition, the prior art describes that yeasts and strains of the genus *Bacillus* can improve the quality of fermented products.

The article of Chi Chun-Hua et al. (Improvement of bioactivity of soybean meal by solid-state fermentation with Bacillus amyloliquefaciens versus Lactobacillus spp. and Saccharomyces cerevisiae, LWT - Food Science and Technology, Volume 68, May 2016, Pages 619-625) teaches, for example, that *Bacillus amyloliquefaciens* U304 and *S*. *cerevisiae* CJ1697 can decompose carbohydrate into carbon dioxide gas, reduce the total amount of carbohydrate and increase the relative protein content in fermented soybean meal.

KR20140144329 discloses a culture method, which uses *Saccharomyces Cerevisiae* MF 10003 strain (KFCC11546P) showing a fermented flavor so as to irradiate fruit flavors to increase preferences of livestock.

However, none of these documents discloses the method as described hereinabove for preparing a fermented product with improved odor, nor the use of yeasts producing α-galactosidase, protease, and phytase for improving the odor of a product fermented with a strain of the genus *Bacillus.*

### DISCLOSURE

### Technical Problem

An aspect of the present invention is to provide a method for preparing a fermented composition with improved odor, which includes preparing grain flour; performing primary fermentation of the grain flour using yeast which produces α-galactosidase, protease, and phytase; performing secondary fermentation of the primary fermented product using a strain of the genus *Bacillus;* and obtaining the secondary fermented product.

Another aspect of the present invention is the use of yeast which produces α-galactosidase, protease, and phytase, for improving the odor of a fermented product of Bacillus.

### Technical Solution

To achieve the above objects, an aspect of the present invention provides a method for preparing a fermented composition with improved odor, which includes preparing grain flour; performing primary fermentation of the grain flour using yeast which produces α-galactosidase, protease, and phytase; performing secondary fermentation of the primary fermented product using a strain of the genus *Bacillus;* and obtaining the secondary fermented product.

In preparing grain flour by the above preparation method, the grain flour may contain without limitation any raw materials commonly used in a process of preparing feed. Specifically, the grain flour may contain soybean, soybean meal, corn or corn gluten, *etc.,* more specifically, soybean meal or corn gluten, and even more specifically, both soybean meal and corn gluten, but the grain flour is not limited thereto.

The grain flour may be one which undergoes moisture content adjustment and heat treatment.

It is known that microorganisms require at least a certain level of moisture for their growth and that it is difficult for them to grow at a moisture concentration of 5% to 12%, which is the concentration of moisture contained in raw materials themselves. Additionally, it is known that most enzymes (*e.g.,* glucoamylase, proteases, *etc*.) undergo a decomposition reaction based on hydrolysis, and thus, at least a certain level of moisture content is necessary to enable a smooth enzyme reaction.

Specifically, the adjusted moisture content within the grain flour may be in a range of 30% to 60%, more specifically 35% to 55%, and more specifically 40% to 50%, but the adjusted moisture content is not limited thereto. The composition having the adjusted moisture content in the above range may be advantageous in that it can prevent the reduction of fermentation rate due to a low moisture content and improve the problem of high cost being incurred in the transfer of the raw materials and the drying process after the fermentation, and additionally, the composition may be advantageous from the aspect of heat efficiency.

In particular, the moisture content can affect the degree of increase in the protein content during fermentation of the composition. Specifically, as the low moisture content becomes lower, it becomes more disadvantageous for the growth of a microorganism, and the degree of increase in the protein content of a composition by fermentation may be reduced. Additionally, when the moisture content is excessively high, the drying cost for removing the moisture contained at the end of the fermentation increases, and as a result, the manufacturing cost may increase and the product competitiveness may be reduced.

Meanwhile, the heat treatment process can sterilize harmful microorganisms contained in the raw materials themselves and reduce those materials which inhibit digestibility, such as anti-nutritional factors (*e.g*., trypsin inhibitors present in the soybean meal or corn gluten). Additionally, since corn gluten has low hygroscopicity, it can help to ensure that sufficient moisture is contained in the corn gluten via a heat treatment process after hydrolysis.

The heat treatment may be performed using various methods known in the art, for example, using steam or superheated steam.

Specifically, the heat treatment may be performed at 90°C to 110°C for 20 to 40 minutes, more specifically at 95°C to 105°C for 25 to 35 minutes, and more specifically at 100°C for 30 minutes, but the heat treatment is not limited thereto.

When the heat treatment temperature is low or the treatment time is short, there is a problem in that the sterilization effect of various germs is lowered and the subsequent fermentation process may not proceed smoothly, whereas when the heat treatment temperature is high or the treatment time is long, there is a problem in that the digestibility may be reduced due to denaturation of the proteins in the composition and thus the quality of the final product may be deteriorated.

In the preparation method of the present invention, the primary fermentation is fermenting the grain flour using yeast.

As used herein, the term "yeast" is a generic term for single-celled organisms, which are a group of fungi or mushrooms but which have neither hyphae nor a function of photosynthesis or motility. Yeast itself is used as a cheap fat/protein source and may also be used for fermentation of food or feed. Specifically, the yeast used for the yeast fermentation of the fermented composition may be *Saccharomyces cerevisiae,* but the yeast is not limited thereto.

Since yeast secretes an enzyme capable of decomposing oligosaccharides, it can decompose oligosaccharides in plant raw materials for feed, and in addition, the cell walls of yeast can be served as a useful component for animals, and thus, the method of preparing a fermented composition using the yeast can improve the components and functionality of the plant raw materials.

The yeast of the present invention produces α-galactosidase, protease, and phytase, and may be, more specifically, *Saccharomyces cerevisiae* deposited under Accession Number KCCM12123P or Accession Number KCCM12124P. Generally, not all yeast can produce α-galactosidase, protease, and phytase, but the yeast of the present invention can produce these enzymes and thus has an excellent effect of improving the odors of the fermented product and composition fermented by *Bacillus* fermentation, compared to other yeast species.

The amount of yeast to be inoculated into the fermented composition can be an important factor that affects the fermentation. The amount of yeast inoculation may be such that the number of yeast immediately after inoculation is in a range of 10⁵ CFU/g to 10⁹ CFU/g, and more specifically, 10⁶ CFU/g to 10⁸ CFU/g, but the amount of yeast inoculation is not limited thereto.

When the amount inoculated is too small, the amount of the fermentation liquid of seed bacteria to be consumed is small, but it requires a longer time for the fermentation of the composition. As a result, the fermentation time required for producing a product becomes longer, thereby increasing the likelihood of contamination with various germs. Meanwhile, when the amount inoculated is too large, the fermentation time may be significantly shortened, but there is a disadvantage in that seed bacteria must be provided for inoculation. In particular, since the fermentation performance depends largely on the growth characteristics of the fermentation strains to be used and the type of the fermentation apparatus, one of ordinary skill in the art will be able to appropriately select the amount of inoculation considering the characteristics of these strains at the production stage.

The performing of the primary fermentation of grain flour of the present invention may further include treating the grain flour with an enzyme. More specifically, the step may include adding α-amylase or glucoamylase.

The preparation method of the present invention can increase the water-soluble saccharide content of a raw material via enzyme treatment of grain flour and can concentrate proteins contained therein via yeast fermentation and *Bacillus* fermentation, and thus it is possible to improve functionality of a composition by increasing the ratio of protein content in the grain flour.

The enzyme treatment of grain flour corresponds to decomposing structural carbohydrates.

As used herein, the term "structural carbohydrate" refers to a carbohydrate with low utilization (*e.g.,* cellulose, hemicellulose, pectin, *etc.*) that constitutes the cell walls of plant cells. Structural carbohydrates can inhibit the utilization of raw materials for feedstuffs (*e.g.,* soybean meal, corn gluten, *etc.*) by fermentation microorganisms and can also decrease the absorption rate and digestion rate of livestock. Accordingly, enzyme treatment may be performed so as to improve the rate of substrate utilization by fermentation microorganisms and the absorption rate and digestion rate of livestock.

Specifically, examples of enzymes that can decompose the structural carbohydrates include α-amylase, glucoamylase, cellulase, pectinase, *etc.,* and more specifically, α-amylase or glucoamylase, but the enzymes are not limited thereto.

The enzyme treatment may be performed simultaneously or sequentially in connection with the inoculation of yeast to the grain flour to be fermented, according to the type of the enzyme.

Specifically, whether the enzyme treatment is performed simultaneously or sequentially may vary depending on the type of the enzymes.

Since the activity conditions vary from enzyme to enzyme, the enzyme treatment step and the yeast fermentation step may be performed considering the enzyme activity conditions and yeast fermentation conditions. For example, when thermophilic α-amylase is used, the enzyme activity requires a high temperature condition. Therefore, a heat treatment process and an enzyme treatment step may be performed simultaneously by adding an enzyme before the heat treatment, after the moisture treatment of the grain flour. That is, the yeast fermentation step may be performed after the heat treatment process and the enzyme treatment step.

Meanwhile, when glucoamylase or mesophilic α-amylase is used, the enzyme activity does not require a high temperature condition. Therefore, an enzyme treatment step may be performed after completing moisture treatment and heat treatment. In this case, the enzyme reaction and the yeast fermentation may be performed separately or simultaneously. For example, the enzyme reaction may be performed at 50°C to 70°C for 30 minutes to 1 hour and 30 minutes by adding glucoamylase or mesophilic α-amylase to grain flour, and then yeast fermentation may be performed by inoculation with yeast. Alternatively, for the optimization of the process, the enzyme reaction step and the yeast fermentation step may be performed simultaneously by adding an enzyme and yeast simultaneously.

The performing of the primary fermentation of the grain flour may further include adding glucoamylase to grain flour in an amount of 0.1 wt% to 1.0 wt%, 0.3 wt% to 0.7 wt%, or 0.5 wt%; inoculating a yeast culture in an amount of 1 wt% to 30 wt%, 1 wt% to 20 wt%, 5 wt% to 15 wt%, 8 wt% to 12 wt%, or 10 wt%; or performing anaerobic fermentation for the grain flour, into which a yeast culture is inoculated, at 10°C to 50°C, 20°C to 40°C, 25°C to 35°C, or 30°C for 1 to 10 hours, 2 to 10 hours, 4 to 8 hours, 5 to 7 hours, or 6 hours.

In the preparation method of the present invention, the secondary fermentation step corresponds to a step in which the primary fermented product undergoes further fermentation using a strain of the genus *Bacillus,* and the secondary fermentation is performed in sequential order following the primary fermentation.

*Bacillus,* which is a genus belonging to the family *Bacillus,* and the bacteria of the genus *Bacillus* bacteria are Gram-positive, rod-shaped bacteria. Specifically, the strain of the genus *Bacillus* may be at least one strain selected from the group consisting of *Bacillus subtilis, Bacillus licheniformis, Bacillus toyoi, Bacillus coagulans, Bacillus polyfermenticus,* and *Bacillus amyloliquefaciens,* more specifically, *Bacillus amyloliquefaciens,* and even more specifically, the strain of the genus *Bacillus* may be the *Bacillus amyloliquefaciens* deposited under Accession Number KCCM11471P, but the strain of the genus *Bacillus* is not limited thereto.

The amount of the strain of the genus *Bacillus* to be inoculated into the fermented composition is the same as described above with regard to the case of the amount of yeast inoculation.

*Bacillus* exhibits an acrid flavor by producing ammonia during fermentation, and this acrid flavor may affect the preference of the fermented composition. However, the preparation method of the present invention can improve the preference of a fermented product of *Bacillus* by improving the peculiar odor of the fermented product of *Bacillus* through the sequential fermentation using yeast and *Bacillus.*

Additionally, by sequentially performing the yeast fermentation and the fermentation by the strain of the genus *Bacillus,* the viable cell count in a fermented composition can be significantly increased, and such an increase in the viable cell count can improve the effects of probiotics of the fermented composition.

In the method of preparing a fermented composition of the present invention, a fermented composition can be produced in which the characteristics and advantages of each of yeast and *Bacillus* are combined. In general, it is known that *Bacillus* cannot produce α-galactosidase and thus cannot completely decompose polysaccharides with a glycosidic bond. However, some yeast strains can produce α-galactosidase, and thus the saccharide component of soybean meal or corn gluten, which is difficult to decompose with a strain of the genus *Bacillus* alone, can be decomposed by yeast, and thus the saccharide component of soybean meal or corn gluten can be effectively used as a substrate for growth and metabolism of fermentation strains.

Specifically, the functionality of a fermented composition can be improved by decomposing the oligosaccharides present in grain flour using an oligosaccharide-decomposing enzyme expressed in yeast and by increasing the viable cell count of the yeast itself, thereby increasing the contents of functional components in the yeast. Additionally, the digestion rate and absorption rate of a feed composition can be improved by decomposing the proteins in the grain flour using a protease expressed in *Bacillus.*

In the method of preparing a fermented composition of the present invention, the fermentation may be a solid fermentation or liquid-state fermentation, and specifically, the fermentation may be a solid fermentation.

As used herein, the term "solid fermentation" refers to a method of performing fermented production by a microorganism using a solid-state raw material containing a certain amount of water.

The secondary fermentation of the present invention, after 2 to 10 hours, 4 to 8 hours, 5 to 7 hours, or 6 hours following the yeast inoculation, may further include inoculating a culture of a strain of the genus *Bacillus* in an amount of 1 wt% to 30 wt%, 1 wt% to 20 wt%, 5 wt% to 15 wt%, 8 wt% to 12 wt%, or 10 wt%; and performing an aerobic fermentation at 35°C to 40°C, or 37°C under a humidity of 80% to 100%, 90% to 100%, 93% to 97%, or 95%.

A secondary fermented product which underwent the secondary fermentation step may be one in which the content of a low molecular weight peptide is in a range of 30% to 100%, more specifically 40% to 100%, 50% to 100%, 60% to 100%, 40% to 90%, 50% to 90%, 40% to 80%, 50% to 80%, 40% to 70%, or 50% to 70%.

The "low molecular weight peptide" refers to a peptide having a molecular weight of 30 kDa or less, and more specifically, 0.1 kDa to 30 kDa, 1 kDa to 30 kDa, 0.1 kDa to 20 kDa, 1 kDa to 20 kDa, 0.1 kDa to 10 kDa, 1 kDa to 10 kDa, or 10 kDa or less.

Still another aspect of the present invention provides the use of yeast which produces α-galactosidase, protease, and phytase, for improving the odor of a fermented product of Bacillus

In one aspect which is not part of the present invention, the present disclosure provides a composition for grain fermentation containing the above yeast, and a feed composition containing the above yeast.

In another aspect which is not part of the present invention, the present disclosure provides a fermented composition prepared by the above method.

In another aspect which is not part of the present invention, the present disclosure provides a feed composition containing the above fermented composition.

The α-galactosidase, protease, phytase, fermented product of *Bacillus,* and yeast are as described above.

The yeast produces α-galactosidase, protease, and phytase, and may be, more specifically, *Saccharomyces cerevisiae* deposited under Accession Number KCCM12123P or Accession Number KCCM12124P.

As used herein, the term "feed composition" refers to a material which supplies the organic or inorganic nutrients that are necessary to maintain the life of a subject and to raise the subject. The feed composition may contain nutrients (*e.g*., energy, proteins, lipids, vitamins, minerals, *etc.*) that are needed by a subject consuming the feed, and may be used as a plant feed (*e.g.,* grains, root meals, by-products of food processing, seaweeds, fibers, fats and oils, starch, gourds, by-products of grains, *etc*.) or an animal feed (*e.g.,* proteins, inorganic matters, fats and oils, minerals, single-cell proteins, zooplanktons, fish meal, *etc.*), but the use of the feed composition is not particularly limited thereto. In the present disclosure, the feed composition is a concept which includes all of the materials that are added to the feed (*i.e.,* feed additive), raw materials for the feed, or the feed itself supplied to the subject.

The subject, which refers to those for raising, may be included without limitation as long as they are organisms that can ingest the feed as described herein. As such, the feed composition as described herein may be applied to a plurality of diets (*i.e.,* feed) for animals including mammals, poultry, fish, and crustaceans. It may be used in commercially important mammals (*e.g.,* pigs, cattle, goats, *etc.*)*,* zoo animals (*e.g.,* elephants, camels, *etc.*), or domestic animals (*e.g.,* dogs, cats, *etc.*)*.* Commercially important poultry may include chickens, ducks, geese, *etc.,* and commercially-raised fish and crustaceans (*e.g*., trout and shrimp) may also be included.

The content of the soybean meal or corn gluten within a feed composition as described herein may be adjusted appropriately according to the kind and age of animals it is to be applied to, application form, desired effects, *etc.,* for example, in a range of 1 wt% to 99 wt%, specifically 10 wt% to 90 wt%, and more specifically 20 wt% to 80 wt%, but the content of the soybean meal or corn gluten is not limited thereto.

For administration, the feed composition as described herein may further include a mixture of at least one of an organic acid (*e.g.,* citric acid, fumaric acid, adipic acid, lactic acid, *etc*.); phosphate (*e.g.,* potassium phosphate, sodium phosphate, polyphosphate, *etc*.); a natural antioxidant (*e.g*., polyphenol, catechin, tocopherol, vitamin C, green tea extract, chitosan, tannic acid, *etc*.); in addition to the hydrolyzed soy protein concentrate. If necessary, other typical additives (*e.g*., an anti-influenza agent, a buffer, a bacteriostatic agent, *etc.*) may be added. In addition, a diluent, a dispersing agent, a surfactant, a binder, or a lubricant may be additionally added to formulate the composition into an injectable preparation (*e.g.,* an aqueous solution, a suspension, an emulsion, *etc.*), a capsule, granule, or a tablet.

Additionally, the feed composition as described herein may be used together with various auxiliary components (*e.g*., amino acids, inorganic salts, vitamins, antioxidants, antifungal agents, antibacterial agents, *etc*.)*,* and a nutrient supplement, a growth accelerator, a digestion/absorption accelerator, and a prophylactic agent, in addition to the main ingredients including a vegetable protein feed (*e.g.,* pulverized or fragmented wheat, barley, corn, *etc*.), an animal protein feed (*e.g.,* blood meal, meat meal, fish meal, *etc.*), animal fat, and vegetable oil.

When the feed composition as described herein is used as a feed additive, the feed composition may be added as it is or used together with other components, and may be appropriately used according to the conventional method. The feed composition may be prepared in the administration form of an immediate-release formulation or sustained-release formulation, in combination with a non-toxic pharmaceutically acceptable carrier. The edible carrier may be corn starch, lactose, sucrose, or propylene glycol. The solid carrier may be in the administration form of tablets, powders, troches, *etc.,* and the liquid carrier may be in the administration form of syrups, liquid suspensions, emulsions, solutions, *etc.* In addition, the administration agent may include a preservative, a lubricant, a solution accelerator, or a stabilizer and may also include other agents for improving inflammatory diseases and a material useful for protection against viruses.

The feed composition as described herein may be mixed in an amount of approximately 10 g to 500 g, specifically 10 g to 100 g per 1 kg, based on the dry weight of the livestock feed, and after being completely mixed, the feed composition may be provided as mash or subjected to a further process of pelletizing, extensification, or extrusion, but is not limited thereto.

According to the method of the present invention described above, it is possible to increase the water-soluble saccharide content of a raw material via enzyme treatment of grain flour and to concentrate the proteins contained therein via yeast fermentation and *Bacillus* fermentation, and thus it is possible to improve the functionality, digestibility, and absorption rate of a composition by increasing the ratio of protein content and the viable cell count in the grain flour.

Additionally, as described above, a fermented product of *Bacillus* produces a peculiar odor during the fermentation process due to ammonia, *etc.* and this may cause a problem in using the corresponding fermented product as feed, *etc.* The yeast as used in the present invention is used in a complex fermentation associated with *Bacillus* fermentation and is thus able to significantly reduce the odor of the product fermented by the *Bacillus* fermentation. Additionally, the yeast as used in the present invention contains the yeast as described herein and is thus able to provide a composition for grain fermentation and feed composition with improved odor.

### Advantageous Effects of the Invention

The method for preparing a fermented composition of the present invention can increase the water-soluble saccharide content of a raw material via enzyme treatment of grain flour and concentrate proteins contained therein via yeast fermentation using yeast which produces α-galactosidase, protease, and phytase, and thus it is possible to improve functionality of a composition by increasing the ratio of protein content and the viable cell count in the grain flour. In particular, since yeast secretes an enzyme capable of decomposing oligosaccharides, it can decompose oligosaccharides in plant raw materials for feed, and in addition, the cell walls of yeast can be served as a useful component for animals, and thus, the method of preparing a fermented composition using the yeast can improve the components and functionality of the plant raw materials. Additionally, the method for preparing a fermented composition of the present invention further includes *Bacillus* fermentation after the yeast fermentation. Since *Bacillus* produces a protease, the proteins in the fermented composition can be peptidized, and as a result, the protein digestibility and absorption rate of feed can be improved. Furthermore, the method for preparing a fermented composition of the present invention can improve the peculiar odor caused by *Bacillus* fermentation through the sequential fermentation of yeast fermentation with yeast producing α-galactosidase, protease, and phytase and *Bacillus* fermentation, thereby enhancing the preference of the composition.

### BRIEF DESCRIPTION OF THE DRAWINGS

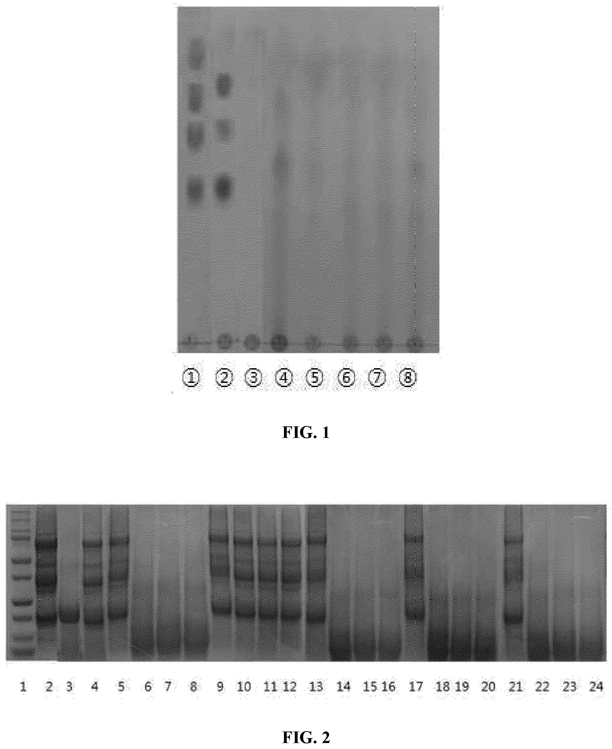

FIG. 1 shows an image illustrating the measurement results of saccharide components in a group of grain raw materials, a group of mixed grains with *Bacillus* fermentation alone, a group of mixed grains with yeast fermentation alone, and a group of mixed grains with combined fermentation by yeast and *Bacillus,* in which (1) represents standard materials: stachyose, raffinose, sucrose, and glucose (from the bottom); (2) represents raw materials of soybean meal; (3) represents raw materials of corn gluten; (4) represents mixed grains (soybean meal + corn gluten) with yeast fermentation alone; (5) represents mixed grains (soybean meal + corn gluten) with yeast (CJN1697) fermentation alone; (6) represents mixed grains (soybean meal + corn gluten) with combined fermentation by yeast (CJN1697) and *Bacillus;* (7) represents mixed grains (soybean meal + corn gluten) with combined fermentation by yeast (CJN2343) and *Bacillus;* and (8) represents mixed grains (soybean meal + corn gluten) with combined fermentation by yeast (Angest^{®}) and *Bacillus.*
FIG. 2 shows an image illustrating the analysis results of protein components for each of the following groups: the group of grain raw materials (soybean meal, corn gluten, and mixed grain raw materials); the group of the grain raw materials with *Bacillus* fermentation alone; the group of the grain raw materials with yeast fermentation alone; and the group of the grain raw materials with combined fermentation by yeast and *Bacillus.*
FIG. 3 shows a chart illustrating the results of the phylogenetic analysis of CJN1697 strain.
FIG. 4 shows a chart illustrating the results of the phylogenetic analysis of CJN2343 strain.

### DETAILED DESCRIPTION OF THE INVENTION

Hereinafter, the present invention will be described in detail through exemplary embodiments. However, these exemplary embodiments are for illustrative purposes only and are not intended to limit the scope of the present invention.

### Example 1: Screening of yeast strains

Among the yeast strains, those strains with excellent abilities of producing α-galactosidase, protease, and phytase were selected. To confirm the ability of producing α-galactosidase, the X-gal agar medium (NaCl (0.5%), peptone (1%), raffinose (1%), agar (1.5%), and X-gal (0.5%)) was prepared. Additionally, to confirm the ability of producing protease, the YM agar medium (powdered skim milk (2%), yeast extract (0.3%), malt extract (0.3%), peptone (1%), and agar (1.5%)) were prepared, and to measure the phytase activity, the medium was prepared by adding phytin to the above medium.

Each yeast strain was cultured in the YPD medium (glucose (2%), yeast extract (0.8%), and soy peptone (0.2%)) at 30°C for 12 hours and thereby the *Saccharomyces cerevisiae* strain was prepared. The prepared yeast culture (5 µL) was added dropwise to each agar medium, cultured at 30°C for about 24 hours, and the abilities of producing α-galactosidase, protease, and phytase were measured by the clear zones generated where each drop of the yeast culture was placed.

The presence of α-galactosidase was confirmed through the X-gal agar medium, and the abilities of producing protease and phytase were examined by measuring and comparing the size of each colony and the clear zone generated around the colony (clear zone size/colony size) (Table 1). In the case of strains where no clear zone generation was observed due to the absence of protease activity, these strains were indicated as "0". As a result, among the about 100 *Saccharomyces cerevisiae* strains, 14 strains were shown to have the α-galactosidase activity, and among the 14 strains, two strains *(i.e.,* CJN1697 and CJN2343) were ultimately selected by excluding those strains where the protease activity necessary for grain fermentation is not present at all.

**Table 1**

| **Strain No.** | **Presence of α-galactosidase** | **Ratio (Clear Zone/Colony Size)** | | **Strain No.** | **Presence of α-galactosidase** | **Ratio (Clear Zone/Colony Size))** | |
|---|---|---|---|---|---|---|---|
| | | **Protease** | **Phytase** | | | **Protease** | **Phytase** |
| CJN1023 | X | 1.11 | 1.58 | CJN2225 | X | 1.03 | 1.32 |
| CJN1025 | X | 1.51 | 1.11 | CJN2229 | X | 1.03 | 1.20 |
| CJN1102 | X | 1.07 | 1.24 | CJN2343 | O | 1.13 | 1.25 |
| CJN1103 | X | 1.19 | 1.29 | CJN2347 | X | 1.11 | 1.33 |
| CJN1105 | X | 1.22 | 1.48 | CJN2350 | X | 1.08 | 1.34 |
| CJN1108 | X | 1.11 | 1.25 | CJN2387 | X | 1.07 | 1.41 |
| CJN1110 | X | 1.11 | 1.33 | CJN2389 | X | 1.09 | 1.42 |
| CJN1145 | X | 1.03 | 1.32 | CJN2390 | X | 1.08 | 1.39 |
| CJN1146 | X | 1.02 | 1.43 | CJN2391 | X | 1.05 | 1.37 |
| CJN1147 | X | 1.00 | 1.58 | CJN2393 | X | 1.11 | 1.32 |
| CJN1148 | X | 1.00 | 1.32 | CJN2394 | X | 1.10 | 1.41 |
| CJN1292 | X | 1.21 | 1.42 | CJN2395 | X | 1.10 | 1.39 |
| CJN1293 | X | 1.00 | 1.52 | CJN2560 | O | 0.00 | 1.09 |
| CJN1294 | X | 1.13 | 1.31 | CJN2562 | O | 0.00 | 1.06 |
| CJN1295 | X | 1.14 | 1.59 | CJN2563 | O | 0.00 | 1.03 |
| CJN1297 | X | 1.10 | 1.38 | CJN2564 | O | 0.00 | 1.13 |
| CJN1298 | X | 1.19 | 1.71 | CJN2565 | O | 0.00 | 1.18 |
| CJN1334 | X | 1.23 | 1.14 | CJN2597 | O | 0.00 | 1.17 |
| CJN1336 | X | 1.00 | 1.24 | CJN2600 | O | 0.00 | 1.23 |
| CJN1437 | X | 1.00 | 1.33 | CJN2601 | O | 0.00 | 1.16 |
| CJN1439 | X | 1.00 | 1.11 | CJN2602 | O | 0.00 | 1.10 |
| CJN 1440 | X | 1.12 | 1.65 | CJN2603 | X | 0.00 | 1.19 |
| CJN1441 | X | 1.02 | 1.43 | CJN2604 | O | 0.00 | 1.10 |
| CJN1442 | X | 1.10 | 1.66 | CJN2605 | O | 0.00 | 1.13 |
| CJN1443 | X | 1.09 | 1.30 | CJN2606 | O | 0.00 | 1.13 |
| CJN1697 | O | 1.02 | 1.50 | CJN2642 | X | 0.00 | 1.03 |
| CJN1837 | X | 1.00 | 1.29 | CJN2643 | X | 0.00 | 1.19 |
| CJN1838 | X | 1.06 | 1.41 | CJN2644 | X | 0.00 | 1.11 |
| CJN1839 | X | 1.04 | 1.41 | CJN2645 | X | 0.00 | 1.15 |
| CJN 1840 | X | 1.03 | 1.37 | CJN2646 | X | 0.00 | 0.00 |
| CJN1841 | X | 1.02 | 1.41 | CJN2647 | X | 0.00 | 1.26 |
| CJN1842 | X | 1.09 | 1.39 | CJN2648 | X | 0.00 | 1.17 |
| CJN1843 | X | 1.09 | 1.26 | CJN2649 | X | 0.00 | 1.13 |
| CJN1844 | X | 1.06 | 1.37 | CJN2650 | X | 0.00 | 1.00 |
| CJN1845 | X | 1.06 | 1.24 | CJN2651 | X | 0.00 | 1.12 |
| CJN 1846 | X | 1.04 | 1.37 | CJN2672 | X | 0.00 | 1.09 |
| CJN 1907 | X | 1.55 | 1.12 | CJN2673 | X | 0.00 | 1.06 |
| CJN 1973 | X | 1.05 | 1.23 | CJN2674 | X | 0.00 | 1.09 |
| CJN1974 | X | 0.00 | 1.29 | CJN2675 | X | 0.00 | 1.12 |
| CJN1975 | X | 0.00 | 1.19 | CJN2676 | X | 0.00 | 1.10 |
| CJN1976 | X | 0.00 | 1.23 | CJN2677 | X | 0.00 | 1.09 |
| CJN2022 | X | 0.00 | 1.33 | CJN2678 | X | 0.00 | 1.14 |
| CJN2023 | X | 0.00 | 1.20 | CJN2679 | X | 0.00 | 1.10 |
| CJN2024 | X | 0.00 | 1.32 | CJN2680 | X | 0.00 | 1.11 |
| CJN2025 | X | 0.00 | 1.35 | CJN2681 | X | 0.00 | 1.18 |
| CJN2026 | X | 0.00 | 1.29 | CJN2682 | X | 0.00 | 1.11 |
| CJN2027 | X | 0.00 | 1.36 | CJN2683 | X | 0.00 | 1.09 |
| CJN2028 | X | 0.00 | 1.29 | CJN2684 | X | 0.00 | 1.10 |
| CJN2029 | X | 1.05 | 1.24 | CJN2685 | X | 0.00 | 1.06 |
| CJN2030 | X | 0.00 | 1.14 | CJN2686 | X | 0.00 | 1.17 |
| CJN2031 | X | 1.03 | 1.57 | CJN2687 | X | 0.00 | 1.12 |
| CJN2067 | X | 1.10 | 1.45 | CJN2688 | X | 0.00 | 1.20 |
| CJN2145 | X | 1.09 | 1.42 | CJN2689 | X | 0.00 | 1.16 |
| CJN2147 | X | 1.03 | 1.22 | CJN2690 | X | 0.00 | 1.03 |
| CJN2150 | X | 1.03 | 1.38 | | | | |

### Example 2: Preparation of fermented composition

### 2-1. Method of moisture treatment and heat treatment of grain flour

Soybean meal flour and corn gluten flour were each prepared. Water was added to the soybean meal to adjust the moisture content of the soybean meal to about 45% based on the weight of the soybean meal and the mixture was subjected to heat treatment at 100°C for 30 minutes. For the corn gluten flour, phosphoric acid was added in an amount of 1.5 wt% based on the weight of the corn gluten, and then water was added to adjust the moisture content of the corn gluten to about 43%, and the mixture was subjected to heat treatment at 100°C for 30 minutes. Sodium hydroxide (NaOH) in an amount of 2.4 wt% based on the weight of the corn gluten was added to the corn gluten, which has undergone heat treatment, and then water was added to adjust the moisture content of the corn gluten to about 45%.

### 2-2. Method of enzyme treatment and preparation of group with yeast fermentation

The soybean meal flour and corn gluten flour prepared by the method of Example 2-1 were mixed at the same weight ratio to prepare mixed grain flour. Then, glucoamylase (0.5 wt%) was added to the mixed grain flour and inoculated with each culture of three kinds of *Saccharomyces cerevisiae* (CJN1697, CJN2343, and commercial bread yeast (purchased from Angel Yeast Co., Ltd.)) in an amount of 10 wt% and mixed, and this was allowed to ferment anaerobically at 30°C for 6 hours, and thereby groups with yeast fermentation were prepared.

### 2-3. Preparation method of group with yeast and Bacillus fermentation

The group of yeast fermentation prepared by the method of Example 2-2 was further subjected to *Bacillus* fermentation. More specifically, as described in Example 2-2, each fermentation group, in which fermentation was performed using the three kinds of *Saccharomyces cerevisiae* (CJN1697, CJN2343, and commercial bread yeast), and 6 hours after the yeast inoculation, was inoculated with the culture of *Bacillus amyloliquefaciens* (KCCM11471P) in an amount of 10 wt%, and then aerobic fermentation was performed in a thermo-hygrostat (temperature: 37°C, humidity: 95%) for 24 hours.

### 2-4. Measurement of components in each experimental group

For each experimental group prepared by the methods of Examples 2-2 and 2-3, the moisture content, viable cell count of *Bacillus,* viable cell count of yeast, and protein content were measured according to time. The protein content was measured by a Kjeldahl apparatus after drying and the fermented product was pulverized. The measured results are shown in Table 2 below.

**Table 2**

| **Sample** | **Time (hr)** | **Moisture Content (%)** | ***Bacillus* (CFU/g)** | **Yeast (CFU/g)** | **Protein Content (Dry Weight, %)** | **Increased Level of Protein Content (%) *Δ*CP** |
|---|---|---|---|---|---|---|
| Group with Yeast Fermentation (CJN1697) | 0 | | | 1.9 × 10⁷ | 61.90 | 1.15 |
| | 6 | 50.57 | | 7.5 × 10⁷ | 63.03 | 2.29 |
| | 22 | 35.85 | | 6.1 × 10⁸ | 67.00 | 6.26 |
| | 26 | 33.66 | | 7.3 × 10⁸ | 67.31 | 6.56 |
| | 30 | 32.78 | 1.4 × 10⁴ | 6.9 × 10⁸ | 66.26 | 5.51 |
| Group 1 with Yeast and *Bacillus Fermentation* (CJN1697 + *Bacillus amyloliquefaciens* ) | 0 | | | 1.9 × 10⁷ | 61.27 | 0.52 |
| | 6 | 50.66 | 1.1 × 10⁷ | 8.4 × 10⁷ | 63.63 | 2.89 |
| | 22 | 44.21 | 5.9 × 10⁹ | 3.8 × 10⁸ | 69.70 | 8.95 |
| | 26 | 39.50 | 9.0 × 10⁹ | 5.4 × 10⁸ | 69.90 | 9.15 |
| | 30 | 35.61 | 8.6 × 10⁹ | 3.9 × 10⁸ | 70.94 | 10.20 |
| Group 2 with Yeast and *Bacillus* Fermentation (CJN2343 + *Bacillus amyloliquefaciens* ) | 0 | | | 2.1 × 10⁷ | 62.32 | 1.57 |
| | 6 | 50.72 | 1.5 × 10⁷ | 8.3 × 10⁷ | 64.51 | 3.76 |
| | 22 | 46.00 | 3.1 × 10⁹ | 4.2 × 10⁸ | 68.89 | 8.14 |
| | 26 | 38.46 | 6.2 × 10⁹ | 4.5 × 10⁸ | 70.23 | 9.48 |
| | 30 | 34.06 | 9.0 × 10⁹ | 4.7 × 10⁸ | 70.49 | 9.74 |
| Group 3 with Yeast and *Bacillus* | 0 | | | 2.3 × 10⁷ | 61.40 | 0.66 |
| | 6 | 50.53 | 1.1 × 10⁷ | 6.9 × 10⁷ | 64.14 | 3.39 |
| Fermentation (Commercial Bread Yeast + *Bacillus amyloliquefaciens*) | 22 | 48.79 | 1.9 × 10⁹ | 3.7 × 10⁸ | 68.51 | 7.77 |
| | 26 | 42.22 | 7.3 × 10⁹ | 3.2 × 10⁸ | 69.72 | 8.97 |
| | 30 | 38.46 | 1.5 × 10¹⁰ | 2.3 × 10⁸ | 70.86 | 10.12 |

As a result, in all of the experimental groups in which yeast fermentation was performed, the amount of live yeast was increased from 10⁷ CFU/g to 10⁸ CFU/g. Additionally, in all of the experimental groups in which *Bacillus* fermentation was performed, the amount of live *Bacillus* was increased from 10⁹ CFU/g to 10¹⁰ CFU/g. Additionally, it was confirmed that the amount of proteins in the composition increased through yeast fermentation, and it was confirmed that the addition of the *Bacillus* fermentation after the yeast fermentation was more effective in increasing the protein amount.

Accordingly, that is, when the *Bacillus* fermentation is performed on the grain raw materials after the yeast fermentation, the protein content of the grain raw materials can be further increased by *Bacillus* without inhibiting the growth of yeast.

### Example 3: Confirmation of degree of oligosaccharide decomposition by fermentation

Two types of groups of grain raw materials (a group of soybean meal raw materials and a group of corn gluten raw materials), a group of mixed grains (soybean meal + corn gluten) with *Bacillus* fermentation alone, a group of mixed grains (soybean meal + corn gluten) with yeast fermentation alone, and a group of mixed grains (soybean meal + corn gluten) with combined fermentation by yeast and *Bacillus* were prepared, and saccharide components were analyzed for each group.

The group of soybean meal raw materials (FIG. 1 (2)) and the group of corn gluten raw materials (FIG. 1 (3)), which were not pretreated, were prepared. The saccharide component of each group was quantitatively analyzed via thin layer chromatography (TLC). 25 mL of distilled water was added to 1 g of each sample, and the mixture was heated in boiling water for 15 to 20 minutes and extracted by shaking at 37°C for 2 hours. The extract was centrifuged and the supernatant was recovered and used as a TLC sample. 2 µL of the supernatant was spotted on a silica gel TLC plate, dried to a constant level, and developed for 3 hours in the developing solution. After the development was completed, the oligosaccharide and monosaccharide spots were confirmed through color development and drying processes.

The group of mixed grains with *Bacillus* fermentation alone (FIG. 1 (4)) was prepared by mixing the soybean meal flour and the corn gluten flour prepared by the method of Example 2-1 at the same weight ratio, inoculating with the culture of *Bacillus amyloliquefaciens* in an amount of 10 wt% to the mixture, followed by performing aerobic fermentation in a thermo-hygrostat (temperature: 37°C, humidity: 95%) for 24 hours.

The group of mixed grains with yeast fermentation alone (FIG. 1 (5)) was prepared by the method of Example 2-2. For the group of mixed grains with combined fermentation by yeast and *Bacillus,* 3 groups (FIG. 1 (6) (CJN1697 + *Bacillus*), FIG. 1 (7) CJN2343 + *Bacillus,* and FIG. 1 (8) bread yeast + *Bacillus*) were prepared using three kinds of yeast.

The saccharide component of each fermentation group was determined in the same manner for the saccharide component measurement in the group of soybean meal raw materials and the group of corn gluten raw materials. For each fermentation group, 25 mL of distilled water was added to 1 g of each sample, and the mixture was heated in boiling water for 15 to 20 minutes and extracted by shaking at 37°C for 2 hours. The extract was centrifuged and the supernatant was recovered and used as a TLC sample.

In FIG. 1, lane (1) represents saccharide component markers of stachyose, raffinose, sucrose, and glucose, in the order of from bottom to top; lane (2) represents the group of soybean meal raw materials; lane (3) represents the group of corn gluten raw materials; lane (4) represents mixed grains (soybean meal + corn gluten) with yeast fermentation alone; lane (5) represents mixed grains (soybean meal + corn gluten) with yeast (CJN1697) fermentation alone; each of lanes (6) to (8) represents the group of mixed grains (soybean meal + corn gluten) with combined fermentation by yeast and *Bacillus* ((6) CJN1697 + *Bacillus*, (7) CJN2343 + *Bacillus*, and (8) bread yeast + *Bacillus*).

Referring to FIG. 1, in the group of single fermentation using *Bacillus* (FIG. 1 (4)), it was confirmed that the saccharide component contained in the soybean meal was not completely decomposed because the microorganism could not sufficiently utilize the saccharide component during the fermentation process, and thus oligosaccharides were included in the fermented product. Meanwhile, in the group where fermentation was performed using yeast (FIG. 1 (5)), it was confirmed that the oligosaccharides were decomposed by yeast and the microorganism had sufficiently utilized the decomposed saccharide component, thus resulting in a low content of oligosaccharides.

Additionally, in the fermentation groups where CJN1697 and CJN2343 yeast strains were used (lanes (6) and (7)), no specific oligosaccharide spot was observed in the fermented product due to the α-galactosidase activity of the enzyme, whereas in the fermentation group where commercial bread yeast (Angest^{®}) was used (lane (8)), the α-galactosidase activity of the bread yeast was low and thus a specific oligosaccharide spot was observed.

Accordingly, the fermented composition prepared using yeast is characterized in that oligosaccharides are decomposed within the composition, and thus the fermented composition does not require any individual digestive enzyme for their decomposition when ingested by an animal through feed, thus making the digestion of feed easier. In particular, it was confirmed that it is more effective to use a yeast strain of CJN1697 or CJN2343.

### Example 4: Confirmation of degree of protein decomposition by fermentation

In the same manner as in Example 3, groups of grain raw materials (soybean meal, corn gluten, and mixed grain raw materials), a group of grain raw materials with *Bacillus* fermentation alone, a group of grain raw materials with yeast fermentation alone, and a group of grain raw materials with combined fermentation by yeast and *Bacillus* were prepared, respectively, and analysis of the protein components in each group was attempted.

As the group of grain raw materials, a total of three kinds of groups (*i.e.,* a group of soybean meal raw materials (group 2), a group of corn gluten raw materials (group 3), and a group of mixed grains in which soybean meal and corn gluten were mixed at the same weight ratio (group 4) were prepared. The protein molecular weight pattern of each raw material was confirmed by SDS-PAGE. 100 mg of each sample was mixed with 5 mL of an 8 M urea solution, and the mixture was sonicated for extraction and centrifuged, and the supernatant was recovered. In each supernatant, protein content was quantitated using bicinchoninic acid and confirmed by SDS-PAGE by loading a certain amount of each protein sample.

The groups of grain raw materials with *Bacillus* fermentation alone (groups 5 to 8) were prepared by inoculating a flour mixture, in which the soybean meal flour and corn gluten flour prepared by the method of Example 2-1 were mixed at the same weight ratio, with the culture of *Bacillus amyloliquefaciens* in an amount of 10 wt%, followed by performing aerobic fermentation in a thermo-hygrostat (temperature: 37°C, humidity: 95%) for 24 hours. The fermentation time (0 hours, 16 hours, 20 hours, and 24 hours) for each group is shown in Table 3 below.

The groups of grain raw materials with yeast fermentation alone (groups 9 to 12) were prepared by the method of Example 2-1, and the groups of grain raw materials with combined fermentation by yeast and *Bacillus* (groups 13 to 24) were prepared by the method of Example 2-3 using three kinds of yeast. The strains used in the fermentation of each group and the fermentation time of each group are shown in Table 3 below. In the groups with combined fermentation where *Bacillus* fermentation proceeded after yeast fermentation, *Bacillus* fermentation proceeded 6 hours after yeast fermentation.

The protein decomposition level of each fermentation group was confirmed by SDS-PAGE. The samples were pretreated in the same manner as in confirming the distribution level of molecular weight of proteins in groups of grain raw materials. The protein molecular weight pattern of each raw material was confirmed by SDS-PAGE. 100 mg of each sample was mixed with an 8 M urea solution, and the mixture was sonicated for extraction and centrifuged, and the supernatant was recovered. The protein content was quantitated using bicinchoninic acid and confirmed by SDS-PAGE by loading a certain amount of each protein sample. The results are shown in FIG. 2.

**Table 3**

| **Experimental group** | **Grain Raw Material** | **Yeast Fermentation** | ***Bacillus* Fermentation** | **Total Fermentation Time (Yeast Fermentation Time + *Bacillus* Fermentation Time)** |
|---|---|---|---|---|
| Group 1 | - | - | - | - |
| Group 2 | Soybean Meal | - | | - |
| Group 3 | Corn Gluten | - | | - |
| Group 4 | Mixed Grain | - | | - |
| Group 5 | Mixed Grain | - | *B. amyloliquefaciens* | 0 |
| Group 6 | Mixed Grain | - | *B. amyloliquefaciens* | 16 |
| Group 7 | Mixed Grain | - | *B. amyloliquefaciens* | 20 |
| Group 8 | Mixed Grain | - | *B. amyloliquefaciens* | 24 |
| Group 9 | Mixed Grain | CJN1697 | - | 6 |
| Group 10 | Mixed Grain | CJN1697 | - | 22 |
| Group 11 | Mixed Grain | CJN1697 | - | 26 |
| Group 12 | Mixed Grain | CJN1697 | - | 30 |
| Group 13 | Mixed Grain | CJN1697 | *B. amyloliquefaciens* | 6 (6 + 0) |
| Group 14 | Mixed Grain | CJN1697 | *B. amyloliquefaciens* | 22 (6 + 16) |
| Group 15 | Mixed Grain | CJN1697 | *B. amyloliquefaciens* | 26 (6 + 20) |
| Group 16 | Mixed Grain | CJN1697 | *B. amyloliquefaciens* | 30 (6 + 24) |
| Group 17 | Mixed Grain | CJN2343 | *B. amyloliquefaciens* | 6 (6 + 0) |
| Group 18 | Mixed Grain | CJN2343 | *B. amyloliquefaciens* | 22 (6 + 16) |
| Group 19 | Mixed Grain | CJN2343 | *B. amyloliquefaciens* | 26 (6 + 20) |
| Group 20 | Mixed Grain | CJN2343 | *B. amyloliquefaciens* | 30 (6 + 24) |
| Group 21 | Mixed Grain | Bread Yeast | *B. amyloliquefaciens* | 6 (6 + 0) |
| Group 22 | Mixed Grain | Bread Yeast | *B. amyloliquefaciens* | 22 (6 + 16) |
| Group 23 | Mixed Grain | Bread Yeast | *B. amyloliquefaciens* | 26 (6 + 20) |
| Group 24 | Mixed Grain | Bread Yeast | *B. amyloliquefaciens* | 30 (6 + 24) |

In Table 3 above, the time of the sequential fermentation groups by yeast and *Bacillus* means the total fermentation time, which is equal to the sum of the fermentation time by *Bacillus* and the yeast fermentation time (*i.e.,* 6 hours).

FIG. 2 shows an image illustrating the SDS-PAGE results of the supernatant proteins in groups 1 to 24.

Referring to Table 3 and FIG. 2, *Bacillus* can produce protease and thus can decompose proteins into low-molecular weight peptides during the fermentation process using the protease. Therefore, in the combined fermentation groups by yeast and *Bacillus,* it was confirmed that the proteins of soybean meal and corn gluten were decomposed. Meanwhile, since yeast cannot produce protease at all, proteins cannot be decomposed at all in the group with yeast fermentation alone, and thus the protein patterns of raw materials were indicated as they were. That is, since the composition which underwent *Bacillus* fermentation after yeast fermentation contains low-molecular weight peptides, the composition can improve the protein absorption rate of feed.

To more specifically measure the content of low-molecular weight peptides in the fermented product, the distribution according to the molecular weight of low-molecular weight peptides was measured using the gel permeation chromatography (GPC) method.

GPC is a method to confirm retention time (RT) by analyzing standard proteins having different molecular weights, and to measure protein distribution of analytes according to molecular weight using molecular weight and a standard curve of RT. To confirm the level of protein decomposition of the raw materials due to fermentation, the protein distribution in the fermented product was analyzed by the GPC method.

GPC analytes were pretreated in the same manner as in the SDS-PAGE method. 100 mg of each sample was suspended in 5 mL of an 8 M urea solvent, and the mixture was sonicated for extraction and centrifuged, and the recovered supernatant was filtered with a syringe filter and used as an analyte for GPC analysis. As the analyte, each of the fermented products from group 4 (mixed raw materials: soybean meal + corn gluten), group 8 (*Bacillus* fermentation alone), group 12 (yeast fermentation alone), and groups 16, 20, and 24 (combined fermentation by *Bacillus* + yeast) was used. The results of GPC analysis are shown in Table 4 below.

**Table 4**

| **Molecular Weight (kDa)** | **Mixed Raw Material** | ***Bacillus* Control** | **Yeast Control** | **CJN1697 + *B*** | **CJN2343 + *B*** | **AngelY + *B*** |
|---|---|---|---|---|---|---|
| | **(Group 4)** | **24 Hr (Group 8)** | **30 Hr (Group 12)** | **30 Hr (Group 16)** | **30 Hr (Group 20)** | **30 Hr (Group 24)** |
| > 75 | 60.11 | 16.38 | 58.36 | 10.51 | 10.36 | 9.50 |
| 30 to 75 | 22.89 | 11.70 | 24.17 | 5.74 | 6.12 | 5.34 |
| 10 to 30 | 7.37 | 21.91 | 7.14 | 16.51 | 17.09 | 16.33 |
| 5 to 10 | 2.78 | 16.20 | 2.72 | 17.93 | 17.90 | 17.75 |
| <5 | 6.86 | 33.81 | 7.61 | 49.31 | 48.53 | 51.07 |
| Total | 100 | 100 | 100 | 100 | 100 | 100 |

Referring to Table 4, the raw material contained more than 82% of polymer peptides of 30 kDa or greater. In the case of *Bacillus* fermentation alone, the content of low-molecular weight peptides of 30 kDa or less was about 71%, whereas in the case of the combined fermentation by *Bacillus* and yeast, the content of low-molecular weight peptides of 30 kDa or less was about 83%, thus showing a significant increase in the content of low-molecular weight peptides. Additionally, when the fermented product was obtained by *Bacillus* and yeast, the content of low-molecular weight peptides of 10 kDa or less within the fermented product was in a range of about 66% to about 69%, thus showing an increase of about 40% compared to the content of low-molecular weight peptides of 10 kDa or less in the product fermented by *Bacillus* fermentation alone. That is, in the case of fermentation by *Bacillus* and yeast, the protein decomposition efficiency was increased and the content of low-molecular weight peptides was increased in the fermented product. Therefore, it can be seen that the digestion and absorption rate can be significantly improved when the product fermented by *Bacillus* and yeast is used as a raw material for food or feed.

### Example 5: Comparison between simultaneous fermentation or sequential fermentation of yeast and Bacillus

The viable cell counts and the amount of protein increase were measured and compared between a case where yeast fermentation and *Bacillus* fermentation are performed simultaneously and a case where yeast fermentation and *Bacillus* fermentation are performed sequentially.

The group with yeast fermentation was prepared by the method of Example 2-2 and the group with sequential fermentation by yeast and *Bacillus* was prepared by the method of Example 2-3. The group with simultaneous fermentation by yeast and *Bacillus* was prepared by mixing the soybean meal flour and the corn gluten flour prepared by the method of Example 2-1 at the same weight ratio, adding glucoamylase (0.5%) thereto, and inoculating simultaneously with yeast and *Bacillus,* followed by performing aerobic fermentation. The group with sequential fermentation by yeast and *Bacillus* was prepared by performing *Bacillus* fermentation 6 hours after yeast fermentation. The moisture content, the viable cell count of *Bacillus,* the viable cell count of yeast, and the amount of proteins were measured according to time in each group, and the results are shown in Table 5 below.

**Table 5**

| **Sample** | **Time (hr)** | **Moisture Content (%)** | ***Bacillus* (CFU/g)** | **Yeast (CFU/g)** | **Protein Content (Dry Weight, %)** | **Increased Level of Protein Content (%) *Δ*CP** |
|---|---|---|---|---|---|---|
| *Bacillus* Control | 0 | 49.6 | 5.5 × 10⁷ | | 61.9 | 2.3 |
| | 16 | 44.6 | 9.6 × 10⁹ | | 69.2 | 9.5 |
| | 20 | 42.4 | 1.3 × 10¹⁰ | | 69.3 | 9.6 |
| | 24 | 38.5 | 9.6 × 10⁹ | | 69.6 | 9.9 |
| Group with Yeast Fermentation (CJN1697) | 0 | 49.1 | | 1.5 × 10⁷ | 61.0 | 1.3 |
| | 16 | 48.5 | | 3.7 × 10⁸ | 67.1 | 7.5 |
| | 20 | 48.8 | | 4.4 × 10⁸ | 68.9 | 9.2 |
| | 24 | 48.4 | | 4.1 × 10⁸ | 67.9 | 8.2 |
| Group with Simultaneous Fermentation by Yeast and *Bacillus* (CJN1697 + *Bacillus amyloliquefaciens* ) | 0 | 49.4 | 4.8 × 10⁷ | 1.7 × 10⁷ | 60.9 | 1.2 |
| | 16 | 41.5 | 1.2 × 10¹⁰ | 3.1 × 10⁷ | 68.0 | 8.4 |
| | 20 | 39.4 | 1.3 × 10¹⁰ | 1.9 × 10⁷ | 69.9 | 10.2 |
| | 24 | 35.8 | 1.1 × 10¹⁰ | 1.6 × 10⁷ | 69.1 | 9.4 |
| Group with Sequential Fermentation by Yeast and *Bacillus* (CJN1697 + *Bacillus amyloliquefaciens* ) | 0 | | | 2.8 × 10⁷ | 60.1 | 0.4 |
| | 6 (6 + 0) | 49.7 | 6.4 × 10⁷ | 3.8 × 10⁷ | 62.4 | 2.7 |
| | 22 (6 + 16) | 45.1 | 6.5 × 10⁹ | 1.9 × 10⁸ | 70.8 | 11.1 |
| | 26 (6 + 20) | 43.3 | 6.9 × 10⁹ | 2.2 × 10⁸ | 715 | 11.9 |
| | 30 (6 + 24) | 40.9 | 1.1 × 10¹⁰ | 1.3 × 10⁸ | 70.7 | 11.0 |

In Table 5 above, the time of the sequential fermentation groups by yeast and *Bacillus* means the total fermentation time which is equal to the sum of the fermentation time by *Bacillus* and the yeast fermentation time (*i.e.,* 6 hours).

Referring to Table 5, when *Bacillus* fermentation was performed following yeast fermentation, both the viable cell count of *Bacillus* and the viable cell count of yeast increased in proportion to their fermentation time. However, it was confirmed that when yeast and *Bacillus* were simultaneously inoculated and fermented together, the viable cell count of *Bacillus* increased in proportion to its fermentation time, but the viable cell count of yeast remained at a level of 10⁷ CFU/g. That is, when yeast and *Bacillus* were simultaneously inoculated and fermented together, yeast did not affect the growth of *Bacillus,* whereas *Bacillus* inhibited the growth of yeast.

Accordingly, it was presumed that the protease of *Bacillus* reduces the population of yeast, which proliferates by budding, and it was confirmed that the order of microbial inoculation has a significant effect for the growth of both microorganisms (*i.e*., yeast and *Bacillus*) in solid fermentation. In particular, it was confirmed that both *Bacillus* and yeast can more readily utilize the water-soluble saccharide component, because the oligosaccharides in the soybean meal are decomposed during yeast fermentation by first performing yeast fermentation for 6 hours.

### Example 6: Problems of odor improvement

A fermented product of *Bacillus* produces a peculiar odor during the fermentation process due to ammonia, *etc.,* and this may be a limiting factor in using feed. Therefore, in this Example, it was confirmed whether or not the complex fermentation by yeast and *Bacillus* reduced the odor of the fermented product of *Bacillus.* An odor test was performed with regard to a mixed raw material of soybean meal and corn gluten, a product fermented by *Bacillus amyloliquefaciens* (KCCM11471P) alone, and a product of complex fermentation by yeast (bread yeast; CJN1697 or CJN2343) and *Bacillus amyloliquefaciens* (KCCM11471P) (50 subjects). The score was determined on a point scale of 0 to 5 such that a higher score represents a higher intensity of peculiar odor of *Bacillus* (Table 6).

As a result, it was confirmed that the product fermented by *Bacillus* fermentation alone (use of *Bacillus amyloliquefaciens* (KCCM11471P)) showed the highest score. Additionally, it was confirmed that when the *Bacillus* sequential fermentation was performed using CJN1697 or CJN2343 yeast, the odor was significantly reduced compared to when the commercially available yeast was used.

**Table 6**

| | **Raw Material** | ***Bacillus* Fermentation Alone** | **Sequential Fermentation of Yeast and *Bacillus* (Bread Yeast)** | **Sequential Fermentation of Yeast and *Bacillus* (CJN1697)** | **Sequential Fermentation of Yeast and *Bacillus* (CJN2343)** |
|---|---|---|---|---|---|
| Mean | 1.6 | 4.75 | 3.8 | 2.5 | 2.45 |
| STDEV | 0.82 | 0.55 | 0.89 | 1.00 | 0.76 |

### Example 7: Analysis of nucleotide sequence of 18S rRNA gene and phylogeny of Saccharomyces cerevisiae strains of the present disclosure

To analyze the strains isolated in Example 1, 18S ribosomal DNA sequencing was performed in the following manner. The chromosomes of CJN1697 and CJN2343 strains were isolated using the Wizard genomic DNA purification kit (Promega, USA), and then subjected to PCR amplification using NS1 (5'-GTAGTCATATGCTTGTCTC-3'; SEQ ID NO: 1) and NS8 (5'-TCCGCAGGTTCACCTACGGA-3'; SEQ ID NO: 2) primers, which are universal primers used in 18S rRNA sequencing. The amplified PCR products were purified using the Wizard SV gel and PCR clean-up system (Promega, USA). As a result, the purified amplified PCR products were compared with the ribosomal DNA sequences of GENEBANK using the BLASTN program, and the sequence homology was compared and analyzed using the Clustal X and Mega 2 programs.

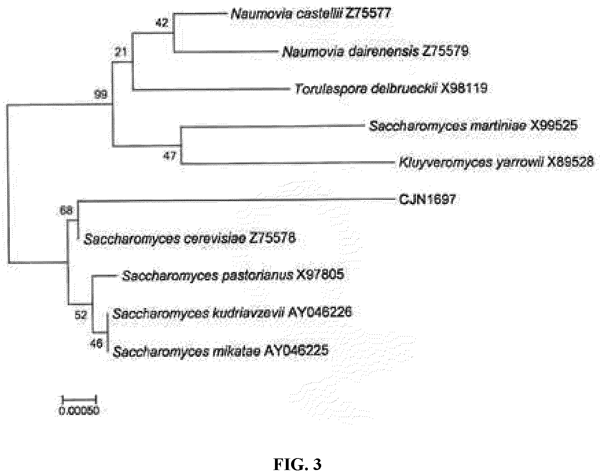

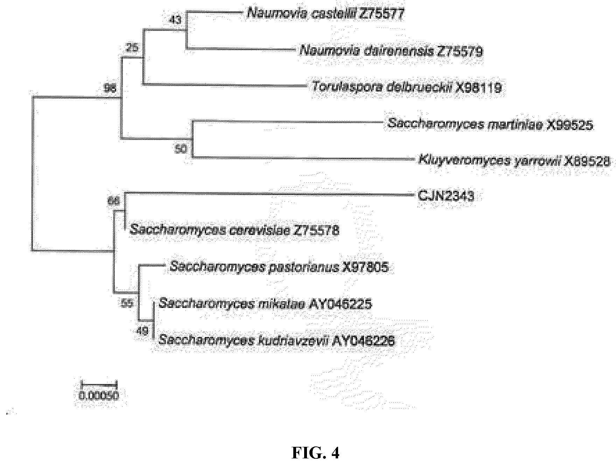

As a result of the phylogenetic analysis, both strains of the present disclosure (*i.e.,* CJN1697 and CJN2343) showed a 99% homology to that of *Saccharomyces cerevisiae*, a reference strain (FIGS. 3 and 4). The strains of the present disclosure (*i.e.,* CJN1697 and CJN2343) were each named *Saccharomyces cerevisiae* CJN1697 and *Saccharomyces cerevisiae* CJN2343, and deposited to the Korean Culture Center of Microorganisms (KCCM) on October 11, 2017, according to the Budapest Treaty under Accession Numbers KCCM12123P and KCCM12124P, respectively.

From the foregoing, a skilled person in the art to which the present disclosure pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that are included within the scope of the present invention as defined by the appended claims.

### ACCESSION NUMBER

Name of Depositary Agency: Korean Culture Center of Microorganisms (KCCM) Deposit Number: KCCM12123P
   Date of Deposition: October 11, 2017
Name of Depositary Agency: Korean Culture Center of Microorganisms (KCCM) Deposit Number: KCCM12124P
   Date of Deposition: October 11, 2017
Name of Depositary Agency: Korean Culture Center of Microorganisms (KCCM) Deposit Number: KCCM11471P
   Date of Deposition: October 11, 2017

## Claims

1. A method for preparing a fermented composition with improved odor, comprising:
- preparing grain flour;
- performing primary fermentation of the grain flour using yeast which produces α-galactosidase, protease, and phytase;
- performing secondary fermentation of the primary fermented product using a strain of the genus *Bacillus;* and
- obtaining the secondary fermented product.

2. The method according to claim **1,** wherein the yeast is *Saccharomyces cerevisiae* deposited under Accession Number KCCM12123P or Accession Number KCCM12124P.

3. The method according to claim **1,** wherein, in the secondary fermented product, a peptide with a molecular weight of 30 kDa or less is contained in an amount of 40% to 100%, wherein said amount is measured using the gel permeation chromatography (GPC) method as defined in the description.

4. The method according to claim **1,** wherein the grain flour comprises soybean meal or corn gluten.

5. The method according to claim **1,** wherein the performing of the primary fermentation of the grain flour comprises adding α-amylase or glucoamylase.

6. The method according to claim **1,** wherein the grain flour undergoes moisture content adjustment and then heat treatment.

7. The method according to claim **6,** wherein the adjusted moisture content is in a range of 30% to 60%.

8. The method according to claim **1,** wherein the strain of the genus *Bacillus* is at least one strain selected from the group consisting of *Bacillus subtilis, Bacillus licheniformis, Bacillus toyoi, Bacillus coagulans, Bacillus polyfermenticus*, and *Bacillus amyloliquefaciens.*

9. The method according to claim **1,** wherein the strain of the genus *Bacillus* is *Bacillus amyloliquefaciens* deposited under Accession Number KCCM114371P.

10. Use of yeast which produces α-galactosidase, protease, and phytase, for improving the odor of a fermented product of *Bacillus.*

## Patentansprüche

1. Verfahren zum Herstellen einer fermentierten Zusammensetzung mit verbessertem Geruch, umfassend:
- Herstellen von Getreidemehl;
- Durchführen von Primärfermentation des Getreidemehls unter Verwendung von Hefe, die α-Galaktosidase, Protease und Phytase erzeugt;
- Durchführen von Sekundärfermentation des primärfermentierten Produkts unter Verwendung eines Stamms der Gattung *Bacillus;* und
- Erhalten des sekundärfermentierten Produkts.

2. Verfahren nach Anspruch **1,** wobei die Hefe *Saccharomyces cerevisiae* ist, hinterlegt unter Hinterlegungsnummer KCCM12123P oder Hinterlegungsnummer KCCM12124P.

3. Verfahren nach Anspruch **1,** wobei in dem sekundärfermentierten Produkt ein Peptid mit einem Molekulargewicht von 30 kDa oder weniger in einer Menge von 40 % bis 100 % enthalten ist, wobei die Menge unter Verwendung des Gelpermeationschromatographie- (GCP-) Verfahrens, wie in der Beschreibung definiert, gemessen wird.

4. Verfahren nach Anspruch **1,** wobei das Getreidemehl Sojamehl oder Maisgluten umfasst.

5. Verfahren nach Anspruch **1,** wobei das Durchführen der Primärfermentation des Getreidemehls das Zugeben von α-Amylase oder Glucoamylase umfasst.

6. Verfahren nach Anspruch **1,** wobei das Getreidemehl eine Feuchtegehaltsanpassung und dann Wärmebehandlung durchläuft.

7. Verfahren nach Anspruch **6,** wobei der angepasste Feuchtegehalt in einem Bereich von 30 % bis 60 % liegt.

8. Verfahren nach Anspruch **1,** wobei der Stamm der Gattung *Bacillus* mindestens ein Stamm ist, ausgewählt aus der Gruppe bestehend aus *Bacillus subtilis, Bacillus licheniformis, Bacillus toyoi, Bacillus coagulans, Bacillus polyfermenticus* und *Bacillus amyloliquefaciens.*

9. Verfahren nach Anspruch **1,** wobei der Stamm der Gattung *Bacillus Bacillus amyloliquefaciens* ist, hinterlegt unter Hinterlegungsnummer KCCM114371P.

10. Verwendung von Hefe, die α-Galactosidase, Protease und Phytase erzeugt, zum Verbessern des Geruchs eines fermentierten Produkts von *Bacillus.*

## Revendications

1. Une méthode pour préparer une composition fermentée avec une odeur améliorée, comprenant :
- préparer de la farine de céréales ;
- réaliser une fermentation primaire de la farine de céréales en utilisant une levure qui produit de l'α-galactosidase, de la protéase et de la phytase ;
- réaliser une fermentation secondaire du produit fermenté primaire en utilisant une souche du genre *Bacillus* ; et
- obtenir le produit fermenté secondaire.

2. La méthode selon la revendication **1,** dans laquelle la levure est *Saccharomyces cerevisiae* déposée sous le numéro d'accession KCCM12123P ou le numéro d'accession KCCM12124P.

3. La méthode selon la revendication **1,** dans laquelle, dans le produit fermenté secondaire, un peptide avec un poids moléculaire de 30 kDa ou moins est contenu dans une quantité de 40 % à 100 %, ladite quantité étant mesurée en utilisant la méthode de chromatographie par perméation de gel (CPG) telle que définie dans la description.

4. La méthode selon la revendication **1,** dans laquelle la farine de céréales comprend de la farine de soja ou du gluten de maïs.

5. La méthode selon la revendication **1,** dans laquelle la réalisation de la fermentation primaire de la farine de céréales comprend l'ajout d'α-amylase ou de glucoamylase.

6. La méthode selon la revendication **1,** dans laquelle la farine de céréales subit un ajustement de la teneur en eau puis un traitement thermique.

7. La méthode selon la revendication **6,** dans laquelle la teneur en eau ajustée se situe entre 30 % et 60 %.

8. La méthode selon la revendication **1,** dans laquelle la souche du genre *Bacillus* est au moins une souche choisie dans le groupe constitué de *Bacillus subtilis, Bacillus licheniformis, Bacillus toyoi, Bacillus coagulans, Bacillus polyfermenticus* et *Bacillus amyloliquefaciens.*

9. La méthode selon la revendication **1,** dans laquelle la souche du genre *Bacillus* est *Bacillus amyloliquefaciens* déposée sous le numéro d'accession KCCM114371P.

10. Utilisation d'une levure produisant de l'α-galactosidase, de la protéase et de la phytase pour améliorer l'odeur d'un produit fermenté de *Bacillus.*
